# FASCICULE DE BREVET EUROPEEN

(11) **EP 1 882 465 B1**
(45) Date de publication et mention de la délivrance du brevet: **07.01.2009**
(21) Numéro de dépôt: 06117726.7
(22) Date de dépôt: 24.07.2006
(51) Int. Cl.: A61F 2/88

(54) **Applicateur d'endoprothèse luminale à action réversible.**
Vorrichtung zum reversiblen Einbringen einer Endoprothese
Reversible-action endoprosthesis delivery device

(43) Date de publication de la demande: 30.01.2008
(73) Titulaire: Cardiatis Société Anonyme, 7000 Mons (BE)
(72) Inventeur: Frid, Noureddine, Beersel, 1650 (BE); Nicaise, Philippe, 1150 Uccle (BE)
(74) Mandataire: Van Straaten, Joop

(56) Documents cités:
- EP-A- 0 829 242
- US-A1- 2004 181 237

## Description

### Domaine de l'invention

L'invention se rapporte aux dispositifs de mise en place d'endoprothèses luminales, tels que des stents, et permet le repositionnement éventuel de ces endoprothèses. L'invention s'applique particulièrement aux endoprothèses auto-expansibles.

### État de la technique

Lors de la mise en place d'une endoprothèse luminale, l'opérateur dépend totalement de l'imagerie médicale. Les faibles dimensions des endoprothèses et le faible contraste des images font qu'il est très difficile, même à un opérateur chevronné, de garantir à 100% un positionnement correct. C'est particulièrement vrai dans le cas d'endoprothèses auto-expansibles, qui ont tendance à se déployer complètement dès leur sortie de l'applicateur et qu'il est donc difficile, voire impossible de repositionner.

On connaît de nombreux applicateurs censés permettre une rétractation suivie d'un repositionnement.

US 5,026,377 montre un applicateur pour stent auto-expansible. L'extrémité proximale du stent est fixée à un poussoir central par un adhésif ou par la présence d'un bourrelet placé sur ce poussoir. US 5,628,755 et US 5,246,421 décrivent des applicateurs pour stents plastiquement déformables (stents à ballon). Dans US 5,628,755, un manchon permet d'ajuster la longueur du ballon à des stents de différentes longueurs.

WO 96/13228 montre un applicateur dote d'un noyau malléable placé sur le poussoir pour le maintien en place d'une endoprothèse avant qu'elle ait été complètement libérée.

WO 99/47075 décrit un applicateur réversible pour endoprothèse comprenant un ballonnet interne qui assure la prise de l'applicateur sur l'endoprothèse tant qu'elle n'a pas été sortie complètement de son logement.

En pratique, aucun de ces applicateurs ne donne des résultats fiables à 100% : dès qu'une longueur relativement limitée du stent est libérée, il est impossible de revenir en arrière pour rectifier sa position. Tous ces dispositifs sont par ailleurs encombrants du fait de la présence du dispositif de retenue à l'intérieur de la gaine, ce qui limite automatiquement le diamètre des vaisseaux où ils peuvent être employés.

On a donc cherché à mettre au point un applicateur peu encombrant, c'est-à-dire occupant un faible diamètre à l'état replié, et permettant une inversion de la pose d'une endoprothèse jusqu'à un stade avancé de la procédure de déploiement.

### Résumé de l'invention

L'objet de l'invention est un applicateur d'endoprothèse luminale à action réversible comprenant: une partie distale et une partie proximale, un poussoir, un fourreau externe coulissant longitudinalement par rapport à ce poussoir. Un organe de retenue constitué d'une tresse expansible s'élargissant en bulbe à l'état expansé, solidaire du poussoir, est disposé longitudinalement à l'intérieur de l'endoprothèse à mettre en place. La partie proximale de l'endoprothèse est pincée entre le bulbe en déploiement et la paroi interne du fourreau, ce qui rend possible la rétractation de l'endoprothèse à l'intérieur du fourreau tant que l'extrémité proximale de l'endoprothèse n'a pas franchi l'extrémité distale du fourreau.

Suivant une forme de réalisation avantageuse l'endoprothèse comprend une armature auto-expansible.

Suivant une autre forme de réalisation avantageuse l'endoprothèse comprend une armature plastiquement déformable.

Suivant une forme de réalisation préférée, la tresse est réalisée en un métal choisi parmi [les alliages de Nickel, les alliages de titane, le Nitinol].

Suivant une forme de réalisation préférée, l'extrémité distale du bulbe est fermée par un sertissage.Ce sertissage peur avantageusement comprendre un marqueur radiopaque.

Le sertissage est percé de préférence d'un orifice permettant le passage d'un guide.

Si l'endoprothèse comprend une armature tressée, la tresse formant le bulbe est constituée de préférence d'un nombre de fil correspondant à celui de l'endoprothèse. Dans ce cas également, les fils de la tresse formant le bulbe ont avantageusement un diamètre sensiblement égal à celui des fils formant l'endoprothèse.

Suivant une forme de réalisation avantageuse, la partie distale du bulbe, à l'état déployé, est rentrée à l'intérieur de la partie proximale du dit bulbe sur tout ou partie de sa longueur de façon à doubler la paroi du dit bulbe.

Un avantage de l'invention est que l'endoprothèse peut être retenue par l'applicateur et recapturée même après que 90% de sa longueur ait été déployée.

Un autre avantage tient à la facilité de mise en place dans l'applicateur tant de l'endoprothèse que de l'organe de retenue.

Un autre avantage réside dans les très faibles dimensions (diamètre) de l'applicateur ainsi équipé, qui peut être introduit en conséquence dans des vaisseaux de très faible diamètre (notamment pour les interventions cervicales).

### Brève description des figures

Ces aspects ainsi que d'autres aspects de l'invention seront clarifiés dans la description détaillée de modes de réalisation particuliers de l'invention, référence étant faite aux dessins des figures, dans lesquelles :
Les Fig. 1 à 4 sont des vues schématiques en élévation avec interruption des différentes étapes de l'introduction d'une endoprothèse avec l'applicateur de l'invention.
Les Fig. 5 à 10 sont des vues schématiques en élévation avec interruption des différentes étapes de mise en place de l'endoprothèse des Fig. 1 à 4.
Les Fig. 11 à 14 sont des vues schématiques en élévation avec interruption de différents modes de réalisation de l'organe de retenue de l'applicateur de l'invention.
Les figures ne sont pas dessinées à l'échelle.

Généralement, des éléments semblables sont dénotés par des références semblables dans les figures.

### Description détaillée des figures

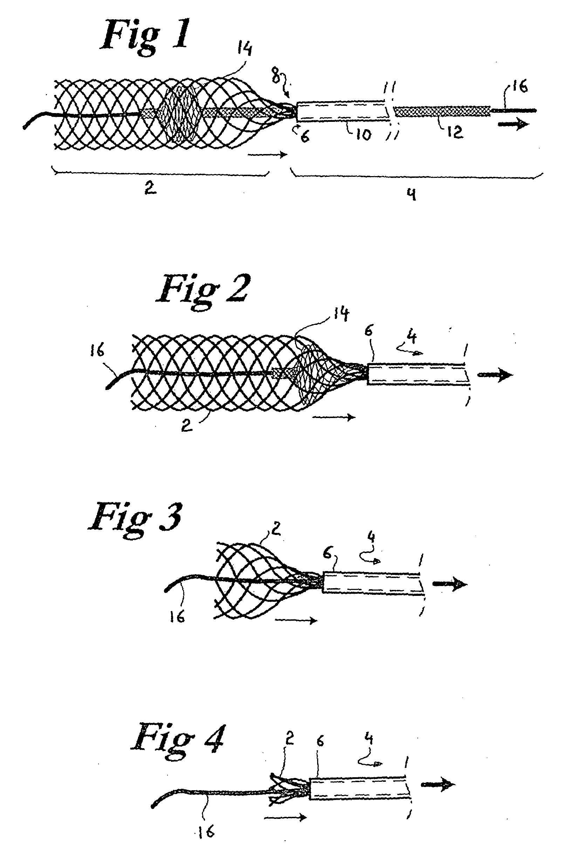

Les Fig. 1 à 4 montrent les différentes étapes de montage d'une endoprothèse 2 (représentée ici sous forme d'un stent) dans l'applicateur de l'invention 4. Seule la partie distale extrême 6 de l'applicateur 4 est représentée.

A la Fig. 1, la partie proximale 8 de l'endoprothèse 2 est comprimée et introduite dans l'extrémité distale 6 d'un fourreau externe 10 qui entoure l'applicateur 4.

Un organe de retenue 12, formé d'une armature tressée se prolongeant à l'intérieur du fourreau 10 par un poussoir 13, est disposé à l'intérieur de l'endoprothèse 2.

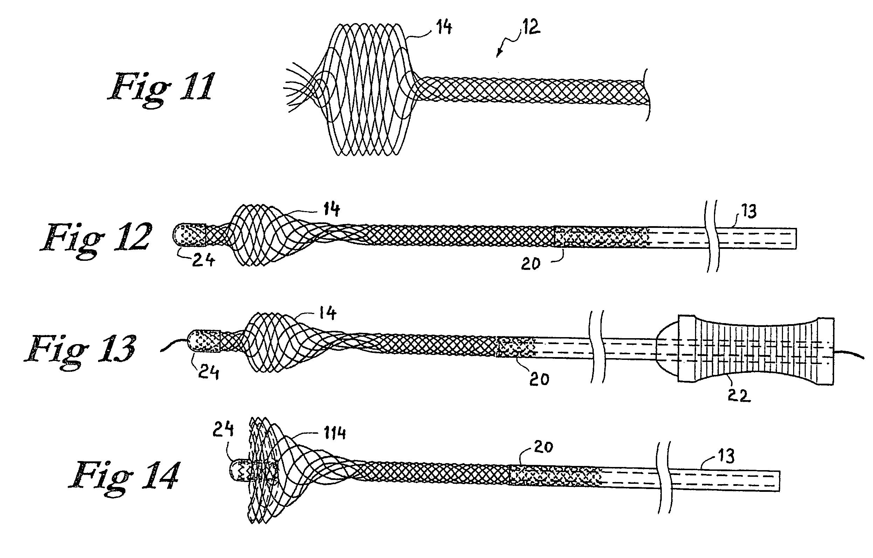

A proximité de l'extrémité distale de cet organe de retenue, l'armature comprend, bien visible à l'état déployé (voir Fig. 11 à 14), un élargissement en bulbe 14. L'ensemble de l'applicateur et les différents organes décrits ci-dessus coulissent le long d'un fil-guide 16 destiné à amener l'extrémité de l'applicateur jusqu'au site de largage de l'endoprothèse.

La Fig. 2 montre ce qui se passe lorsqu'on exerce une traction sur l'organe de retenue depuis la partie proximale de l'applicateur 4 :L'élargissement en bulbe 14 vient s'adosser contre la partie proximale "en entonnoir" de l'endoprothèse 2. La force radiale résultant de la compression du bulbe 14 engendre une force de frottement contre la paroi interne de l'endoprothèse 2. Les différents paramètres régissant cette force de frottement sont calculés de façon à ce que le bulbe entraîne l'endoprothèse 2 et la force à pénétrer dans le fourreau 10 en adoptant une forme contractée.

L'organe de retenue 12 et l'endoprothèse 2 s'enfoncent progressivement à l'intérieur du fourreau (Fig. 2 et 3).

L'organe de retenue 12 étant constitué d'une tresse, on constate qu'à l'état comprimé, les brins formant cette tresse s'alignent à l'intérieur du fourreau 10, si bien que cet organe occupe en définitive une place insignifiante à l'intérieur du fourreau 10. Le diamètre de l'applicateur peut donc être considérablement réduit par rapport à d'autres dispositifs à recapture, de sorte que l'applicateur peut être introduit sans problème dans des vaisseaux sanguins extrêmement fins, et servir donc notamment pour des applications cervicales.

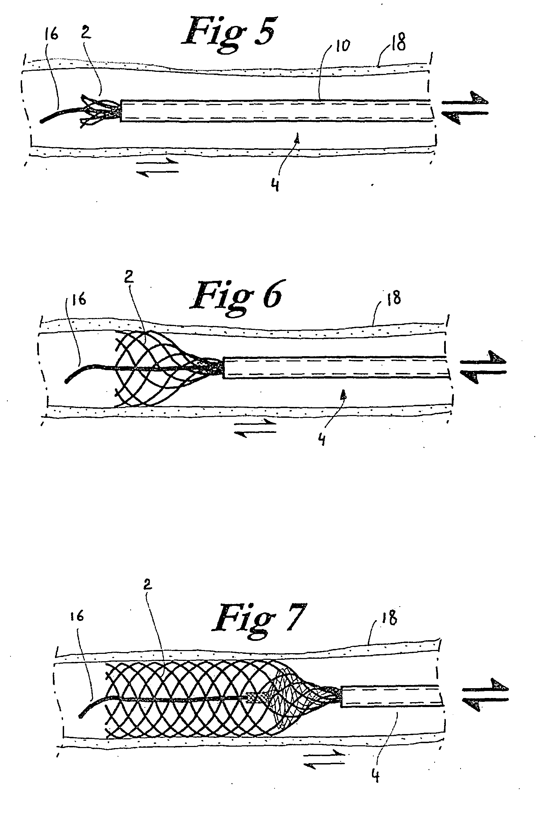

Les Fig. 5 à 10 représentent les différentes étapes de largage in situ d'une endoprothèse, à l'intérieur d'un vaisseau sanguin 18 à traiter.

A la Fig. 5, l'applicateur 4, que l'opérateur a fait coulisser le long du fil-guide 16 depuis un site d'introduction, est arrivé à la hauteur du site à traiter.

L'opérateur, tout en maintenant l'endoprothèse 2 en place grâce au poussoir 13, opère une traction sur le fourreau 10 : l'endoprothèse 2 commence à sortir par l'extrémité distale 6 du fourreau 10. Comme représenté à la Fig. 6, l'endoprothèse 2 (qui est ici un stent auto-expansible), commence à se déployer. Sa paroi latérale vient s'appliquer contre la paroi latérale du vaisseau sanguin 18 de façon à lui restituer son diamètre souhaité. L'opérateur continuant à tirer sur le fourreau 10, le bulbe 14 de l'organe de retenue 12 commence à émerger du fourreau 10.

Jusqu'à ce stade, l'opérateur a la possibilité d'interrompre l'opération de mise en place à tout moment.

S'il estime, au vu des indications qui lui sont transmises par imagerie médicale (rayons X, scanner, etc.) que la position de l'endoprothèse 2 n'est pas idéale, il lui suffit d'inverser le mouvement du fourreau 10. Aussitôt, l'endoprothèse 2, toujours maintenue par frottement par l'organe de retenue 12, reprend sa forme contractée et regagne l'intérieur du fourreau sans abîmer la paroi du vaisseau sanguin, suivant l'ordre (inversé) des Fig. 7-6-5.

La difficulté de positionnement d'une endoprothèse 2 résulte de plusieurs facteurs : d'une part, dans le cas d'une endoprothèse à armature tressée, en fonction du type de maillage et de l'angle de croisement des fils, le coefficient d'allongement de l'endoprothèse 2 peut varier fortement lors du passage de l'état contracté à l'état déployé. D'autre part, la présence de vaisseaux secondaires venant se raccorder au vaisseau traité peut venir compliquer sérieusement la tâche de l'opérateur. En effet, certains types d'endoprothèses 2 risquent d'obturer purement et simplement le débouché de ces vaisseaux secondaires sur le vaisseau traité, avec des conséquences négatives sur l'irrigation de tissus situés en aval de ces vaisseaux.

En fonction du temps dont il dispose, l'opérateur peut donc être amené à procéder à plusieurs essais avant de déterminer l'emplacement le plus adéquat.

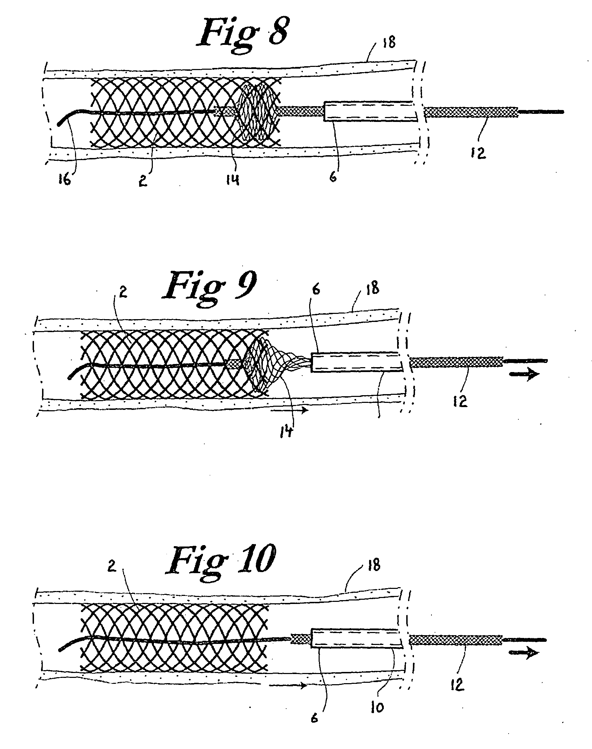

Dès que l'opérateur estime avoir atteint le site idoine, parvenu au stade décrit à la Fig. 7 (qui correspond jusqu'à entre 90 et 95% de la longueur de l'endoprothèse), il peut parachever le processus de déploiement : comme montré à la Fig. 8, le fourreau 10 est tiré vers l'arrière suffisamment pour dégager la partie proximale de l'endoprothèse, laquelle, ainsi totalement libérée, se déploie intégralement le long de la paroi du vaisseau sanguin 18. Il suffit désormais d'un mouvement combiné du poussoir et/ou du fourreau 10 pour faire rentrer le bulbe 14 de l'organe de retenue à l'intérieur du fourreau 10 (Fig. 9) et l'applicateur peut être retiré sans problème (Fig. 10).

Bien que l'applicateur ait été décrit ici dans le cadre de la mise en place d'une endoprothèse auto-expansible (ou d'un stent de même), il va de soi qu'il peut être également utilisé pour la pose d'une endoprothèse à armature à déformation plastique (endoprothèse "à ballonnet").

L'applicateur de l'invention se fait remarquer par son faible diamètre et aussi par l'extrême simplicité de sa manipulation. Il n'est par ailleurs pas nécessaire de disposer d'organe de commande compliqué (air comprimé, etc.) pour l'activer. Sa conception est également très simple dès lors que l'on a déterminé les paramètres « mécaniques » (tels que la force de frottement engendrée) à respecter pour différents types d'endoprothèses. On peut notamment assembler par fusion ou collage 20 le poussoir 13 et l'organe de retenue 12, comme montré aux Fig. 13 à 14. Pour faciliter les manipulations, l'extrémité proximale du poussoir est généralement terminée par un organe de préhension (poignée 22).

Les Fig. 11 à 14 montrent différentes formes de réalisation possibles, mais non limitatives, du bulbe de retenue 14.

A la Fig. 11, le bulbe 14 se termine à son extrémité distale par un simple resserrement, les brins étant par ailleurs laissés libres. Ce mode de réalisation a l'avantage de permettre un encombrement minimum. Toutefois, sa précision est amoindrie.

Le bulbe de la Fig. 12 est fermé à son extrémité distale par un sertissage 24, qui permet un calibrage plus précis de la force radiale engendrée et donc une recapture de l'endoprothèse à un stade très avancé du déploiement. Ce mode de réalisation présente par ailleurs un autre avantage : La faible masse de l'endoprothèse 2 rend normalement son repérage par imagerie médicale très difficile, a fortiori si l'on travaille dans le domaine cervical, à l'intérieur la boîte crânienne. Cependant, l'opérateur peut être aidé dans sa décision par la présence de "marqueurs" radio-opaques qui lui indiquent de façon plus précise la position de l'endoprothèse et/ou de l'extrémité de l'applicateur dans le corps. Pour l'aider dans cette tâche, un repère en matériau radiopaque (platine, or, titane) peut être placé sur le sertissage qui ferme le bulbe. Par ailleurs, le matériau utilisé pour réaliser le sertissage peut lui-même être radiopaque.

Comme le montre la Fig.13, le sertissage 24 est généralement percé d'un orifice permettant le passage d'un fil-guide 16.

Le mode de réalisation du bulbe 114 de la Fig. 14 présente une autre particularité : il est partiellement retourné en corolle du côté distal, ce qui en augmente la force radiale, toutes autres choses (nombre, nature et diamètre des fils) étant par ailleurs égales. On peut également « doubler » le bulbe sur tout ou partie de sa longueur en forçant le sertissage à rentrer plus avant dans le bulbe au moment de sa mise en forme. On notera que même ainsi « doublé », le bulbe ne prend pas davantage de place dans le fourreau externe, étant donné qu'il est complètement étiré au moment de l'insertion dans ce fourreau.

Comme il a été dit plus haut, les paramètres relatifs au bulbe doivent être bien adaptés aux caractéristiques mécaniques de l'endoprothèse 2 qui doit être mise en place. Or, si l'armature de l'endoprothèse est réalisée par tressage, un élément déterminant pour ces caractéristiques mécaniques est le tressage lui-même : son « pas » ainsi que le nombre, la nature et le diamètre des fils. En conséquence, les essais montrent qu'il est particulièrement avantageux d'utiliser pour l'organe de retenue une tresse dont les caractéristiques se rapprochant autant que possible des celles de l'endoprothèse à mettre en place, notamment en ce qui concerne le nombre, voire le diamètre des fils. On peut cependant descendre, pour les fils du bulbe jusqu'à un diamètre qui correspond à seulement 80% de celui des fils de l'endoprothèse, ce qui permet un gain de place et en conséquence une réduction du diamètre de l'applicateur (et, par contrecoup, la possibilité de traiter des vaisseaux sanguins d'encore plus faible diamètre). Il va de soi que l'on peut combiner les différents critères entre eux ou faire intervenir d'autres critères permettant d'obtenir un effet de retenue équivalent, comme la rugosité ou l'état de surface des fils, le « doublage » du bulbe, etc.

Par ailleurs, le diamètre relatif du bulbe joue également un rôle dans la pression qui est appliquée sur la paroi de l'endoprothèse pour la maintenir. Les essais montrent qu'un résultat optimal est atteint lorsque le diamètre du bulbe est compris entre 90 et 95% du diamètre intérieur de l'endoprothèse. Au-delà de 95% la friction risque de devenir trop forte, alors qu'au dessous de 90% on risque un contact insuffisant pour réussir le retrait.

Il apparaîtra évident pour l'homme du métier que la présente invention n'est pas limités aux exemples illustrés et décrits ci-dessus. L'invention comprend chacune des caractéristiques nouvelles ainsi que leur combinaison. La présence de numéros de référence ne peut être considérée comme limitative. L'usage du terme « comprend » ne peut en aucune façon exclure la présence d'autres éléments autres que ceux mentionnés. L'usage de l'article défini « un » pour introduire un élément n'exclut pas la présence d'une pluralité de ces éléments. La présente invention a été décrite en relation avec des modes de réalisations spécifiques, qui ont une valeur purement illustrative et ne doivent pas être considérés comme limitatifs

## Revendications

1. Un applicateur d'endoprothèse luminale (2) à action réversible comprenant : une partie distale et une partie proximale (6), un poussoir, un fourreau externe(10) coulissant longitudinalement par rapport à ce poussoir, un organe de retenue (12) solidaire du poussoir, disposé longitudinalement à l'intérieur de l'endoprothèse (2) à mettre en place, la partie proximale de l'endoprothèse (2) étant pincée entre le dit bulbe (14) et la paroi interne du fourreau (10), de façon à permettre la rétractation de l'endoprothèse (2) à l'intérieur du fourreau (10) tant que l'extrémité proximale de l'endoprothèse (2) n'a pas franchi l'extrémité distale du fourreau (10),
**caractérisé en ce que** l'organe de retenue (12) est constitué d'une tresse expansible s'élargissant en bulbe (14) à l'état expansé.

2. Un applicateur d'endoprothèse selon la revendication 1, **caractérisé en ce que** l'endoprothèse (2) comprend une armature auto-expansible

3. Un applicateur d'endoprothèse selon la revendication 1 **caractérisé en ce que** l'endoprothèse (2) comprend une armature plastiquement déformable.

4. Un applicateur d'endoprothèse selon l'une quelconque des revendications 1 à 3 , **caractérisé en ce que** la tresse formant l'organe de retenue est réalisée en métal choisi parmi [les alliages de Nickel, les alliages de titane, le Nitinol].

5. Un applicateur d'endoprothèse selon l'une quelconque des revendications 1 à 4, **caractérisé en ce que** l'extrémité distale du bulbe (14) est fermée par un sertissage (24).

6. Un applicateur d'endoprothèse selon la revendication 5, **caractérisé en ce que** le sertissage (24) fermant l'extrémité distale du bulbe comprend un marquage radiopaque.

7. Un applicateur d'endoprothèse selon l'une quelconque des revendications 5 ou 6, **caractérisé en ce que** le sertissage (24) est percé d'un orifice permettant le passage d'un guide (16).

8. Un applicateur d'endoprothèse selon l'une quelconque des revendications 1 à 7, **caractérisé en ce que,** si l'endoprothèse (2) comprend une armature tressée, la tresse formant le bulbe (14) est constituée d'un nombre de fil correspondant à celui de l'endoprothèse.

9. Un applicateur d'endoprothèse selon la revendication 8, **caractérisé en ce que** les fils de la tresse formant le bulbe (14) ont un diamètre sensiblement égal à ceux de l'endoprothèse (2).

10. Un applicateur d'endoprothèse selon l'une quelconque des revendications 1 à 9, **caractérisé en ce que** la partie distale du bulbe (14), à l'état déployé, est rentrée à l'intérieur de la partie proximale du dit bulbe sur tout ou partie de sa longueur de façon à doubler la paroi du dit bulbe (14).

## Claims

1. Reversible applicator for an intraluminal endoprosthesis (2), comprising: a distal part and a proximal part (6), a pusher, an outer sheath (10) sliding longitudinally relative to this pusher, a retention element (12) joined to the pusher, arranged longitudinally inside the endoprosthesis (2) that is to be fitted in place, the proximal part of the endoprosthesis (2) being pinched between said bulb (14) and the inner wall of the sheath (10), in such a way as to allow the endoprosthesis (2) to be retracted inside the sheath (10) as long as the proximal end of the endoprosthesis (2) has not passed beyond the distal end of the sheath (10),
**characterized in that** the retention element (12) formed by an expandable braid that widens in a bulb shape (14) in the expanded state,.

2. Endoprosthesis applicator according to Claim 1, **characterized in that** the endoprosthesis (2) comprises a self-expanding framework.

3. Endoprosthesis applicator according to Claim 1, **characterized in that** the endoprosthesis (2) comprises a plastically deformable framework.

4. Endoprosthesis applicator according to any one of Claims 1 to 3, **characterized in that** the braid forming the retention element is made of metal chosen from among [alloys of nickel, alloys of titanium, Nitinol].

5. Endoprosthesis applicator according to any one of Claims 1 to 4, **characterized in that** the distal end of the bulb (14) is closed by an area of crimping (24).

6. Endoprosthesis applicator according to Claim 5, **characterized in that** the area of crimping (24) closing the distal end of the bulb comprises a radiopaque marker.

7. Endoprosthesis applicator according to either of Claims 5 and 6, **characterized in that** the area of crimping (24) has an orifice extending through it to permit passage of a guide (16).

8. Endoprosthesis applicator according to any one of Claims 1 to 7, **characterized in that,** if the endoprosthesis (2) comprises a braided framework, the braid forming the bulb (14) is composed of a number of filaments corresponding to that of the endoprosthesis.

9. Endoprosthesis applicator according to Claim 8, **characterized in that** the filaments of the braid forming the bulb (14) have a diameter substantially equal to those of the endoprosthesis (2).

10. Endoprosthesis applicator according to any one of Claims 1 to 9, **characterized in that** the distal part of the bulb (14), in the deployed state, is drawn back inside the proximal part of said bulb along all or part of its length, in order to double the wall of said bulb (14).

## Patentansprüche

1. Applikator für luminale Endoprothese (2) mit umkehrbarer Wirkung, Folgendes aufweisend: einen distalen Teil und einen proximalen Teil (6), einen Stößel, eine äußere Hülse (10), die entlang dieses Stößels gleitet, ein Rückhalteelement (12), das fest mit dem Stößel verbunden ist, das längs im Inneren der anzubringenden Endoprothese (2) angeordnet ist, wobei der proximale Teil der Endoprothese (2) zwischen dem Wulst (14) und der Innenwand der Hülse (10) derart eingeklemmt ist, dass das Zurückziehen der Endoprothese (2) in das Innere der Hülse (10) erlaubt wird, solange das proximale Ende der Endoprothese (2) das distale Ende der Hülse (10) nicht überschritten hat,
**dadurch gekennzeichnet, dass** das Rückhalteorgan (12) aus einer expandierenden Flechte besteht, die sich im expandierten Zustand wulstförmig (14) verbreitert.

2. Applikator für Endoprothese nach Anspruch 1, **dadurch gekennzeichnet, dass** die Endoprothese (2) eine selbstexpandierende Armierung aufweist.

3. Applikator für Endoprothese nach Anspruch 1, **dadurch gekennzeichnet, dass** die Endoprothese (2) eine verformbare Kunststoffarmierung aufweist.

4. Applikator für Endoprothese nach einem der Ansprüche 1 bis 3, **dadurch gekennzeichnet, dass** die Flechte, die das Rückhalteorgan bildet, aus Metall hergestellt ist, das aus den [Nickellegierungen, Titanlegierungen, Nitinol] ausgewählt wird.

5. Applikator für Endoprothese nach einem der Ansprüche 1 bis 4, **dadurch gekennzeichnet, dass** das distale Ende des Wulstes (14) durch eine Crimpverbindung (24) geschlossen ist.

6. Applikator für Endoprothese nach Anspruch 5, **dadurch gekennzeichnet, dass** die Crimpverbindung (24), die das distale Ende des Wulstes schließt, eine strahlungsundurchlässige Kennzeichnung aufweist.

7. Applikator für Endoprothese nach einem der Ansprüche 5 oder 6, **dadurch gekennzeichnet, dass** die Crimpverbindung (24) mit einer Öffnung durchbohrt ist, die das Durchgehen einer Führung (16) erlaubt.

8. Applikator für Endoprothese nach einem der Ansprüche 1 bis 7, **dadurch gekennzeichnet, dass**, wenn die Endoprothese (2) eine Flechtarmierung aufweist, die Flechte, die den Wulst (14) bildet, aus einer Anzahl von Drähten besteht, die der der Endoprothese entspricht.

9. Applikator für Endoprothese nach Anspruch 8, **dadurch gekennzeichnet, dass** die Drähte der Flechte, die den Wulst (14) bildet, einen Durchmesser im Wesentlichen gleich denen der Endoprothese (2) haben.

10. Applikator für Endoprothese nach einem der Ansprüche 1 bis 9, **dadurch gekennzeichnet, dass** der distale Teil des Wulstes (14) im geöffneten Zustand in das Innere des proximalen Teils des Wulstes über einen Teil oder seine ganze Länge derart eingezogen ist, dass die Wand des Wulstes (14) verdoppelt wird.
